# EUROPEAN PATENT SPECIFICATION

(11) **EP 1 732 479 B1**
(45) Date of publication and mention of the grant of the patent: **27.04.2011**
(21) Application number: 05729016.5
(22) Date of filing: 24.03.2005
(51) Int. Cl.: A61F 2/44

(54) **PROSTHETIC SPINAL DISC**
BANDSCHEIBENPROTHESE
PROTHESE DE DISQUES INTERVERTEBRAUX

(30) Priority: 26.03.2004 GB 0406832; 05.04.2004 GB 0407718; 04.02.2005 GB 0502287
(43) Date of publication of application: 20.12.2006
(73) Proprietor: NuVasive, Inc., San Diego CA 92121 (US)
(72) Inventor: MCLEOD, Alan Rory Mor, Pearsalls Limited, Taunton, Somerset TA1 1RY (GB); REAH, Christopher, Pearsalls Limited, Taunton, Somerset TA1 1RY (GB)
(74) Representative: Merrifield, Sarah Elizabeth
(86) International application number: PCT/GB2005/001161
(87) International publication number: WO 2005/092247

(56) References cited:
- EP-A- 0 453 393
- US-A- 3 867 728
- US-A- 6 093 205
- US-B1- 6 419 704
- US-B1- 6 620 196

## Description

This invention concerns improvements in and relating to surgical implants, particularly, but not exclusively in relation to surgical implants for the replacement of intervetebral discs in the lumbar region of the spine.

Increasingly there is a desire to address problems with intervertebral discs by replacing all or part of the disc with a prosthetic disc rather than fusing the adjacent vertebrae. A wide variety of designs of disc prostheses exist. Generally they are based upon either articulated metal plates or metal end plates with a polyethylene spacer. Generally such devices face problems in terms of the reduced mobility they provide, are reliant upon absolutely correct positioning and do not emulate fully the normal motion they aim to replace.

Previously there has been developed a disc prosthesis including an element of elastomeric or visco-elastic material, the element being provided in a retaining fabric, US-6093205. The disc prosthesis was particularly developed for use in the cervical region of the spine.

US 6,419,704 describes artificial intervertebral disc replacement methods and apparatus. It includes a prostheses with a core of elastomeric material, the core having inner and outer components of fabric.

The present invention has amongst its aims to provide an improved partial or total spinal disc replacement, particularly in the lumbar region. The present invention has amongst is aims to provide a more reliable spinal disc replacement, particularly for the lumbar region,

The present invention provides a disc prosthesis as set out in claim 1.

Various options, possibilities and features of the invention are now provided.

The core may be a single component. The core may be formed of multiple components. A single or multiple components may be provided within an inner component, such as a jacket. Where multiple components are used it is preferred that each is provided within its own inner component. The component(s) may be blocks, beads, spheres, cylinders, rods or other such elements. Multiple component forms for the core are particularly suited to minimally invasive surgical techniques as the core can be formed in the inner component in-situ.

The core may be a continuation of the inner component. For instance, the inner component may be provided with one or more further elements, potentially integral therewith or attached thereto, which form the core. In such an embodiment, the core may be formed by folded and/or bending and/or spiralling one or more further elements within the inner component. The core may be within the inner component by virtue of a the inner component extending for 360 degrees or more about a part of the core, for instance in one or more views. In such embodiments the core may particularly be formed from an octagonal spiral. In such embodiments, the inner may or may not provide a top wall and/or bottom wall to surround the core. In such embodiments, the outer component may be separate from the inner component and its core. The core and inner component in such an embodiment may be formed of different materials and/or formed in different ways and/or be provided with different properties. In particular the core may mimic the properties of the nucleus and the inner component may mimic the properties of the annulus, or properties intermediate the nucleus and annulus. A core provided as a continuation of the inner component may be provided with additional core material, such as an elastomeric material, visco-elastic material and/or hydrogel. Preferably core material in a form which can flow into and around the core and then set is so provided.

The core may be a continuation of the inner component with the inner component itself being a continuation of the outer component. For instance, the outer component may be provided with one or more parts, potentially integral therewith or attached thereto, which form the inner component and/or be provided with one or more further elements, potentially integral therewith or attached thereto, which form the core. In such an embodiment, the core may be formed by folded and/or bending and/or spiralling one or more further elements within the inner component and/or the inner component may be formed by folded and/or bending and/or spiralling one or more parts within the outer component. The core may be within the inner component by virtue of the inner component extending for 360 degrees or more about a part of the core, for instance in one or more views and/or the inner component may be within the outer component by virtue of the outer component extending for 360 degrees or more about a part of the inner component, for instance in one or more views. In such embodiments the core may particularly be formed from an octagonal spiral, with a continuation of the octagonal spiral forming the inner component and a further continuation providing the outer component. The core and/or inner component and/or outer component in such an embodiment may be formed of different materials and/or formed in different ways and/or be provided with different properties. In particular the core may mimic the properties of the nucleus and/or the inner component may mimic the properties of the annulus, or properties intermediate the nucleus and annulus and/or the outer component may mimic properties of the annulus and/or the anterior longitudinal ligament(s). A core provided as a continuation of the inner component and/or outer component and/or an inner component provided as a continuation of the outer component may be provided with additional core material, such as an elastomeric material, visco-elastic material and/or hydrogel. Preferably core material in a form which can flow into and around the core and then set is so provided.

The core may be formed of a single material type or of multiple material types. The core may be an elastomeric material. The core may be a visco-elastic material. The core may be a hydrogel, particularly an elastomeric one. The core may include silicone based materials. The core may include materials having a Shore A hardness of 35 to 80°. The core may be impregnated and/or doped and/or provided with further materials. The further materials may be or include barium sulphate.

The core may be provided of fibrous material, for instance such material provided in a single plane. The fibrous material may be provided with a proportion, preferably the majority, of the fibres at an angle of between 10 and 80 degrees to the horizontal. Such a material may be provided of embroidery and/or other fibrous assembly technique. Preferably such a material resembles the structure and/or properties of the fibrous material of the spine. The core may be formed of a coiled material, particularly a fibrous material. Such a fibrous material may be elastomeric and/or polyester and/or the other fibre materials mentioned herein.

Preferably the core provides equivalent properties and/or behaviour to the nucleus pulposus of a natural disc, for instance during compression and/or distraction and/or horizontal gliding and/or axial rotation and/or flexion and/or extension.

The core may provide a planar top surface and/or planar lower surface. The area of the top surface may be the same as the area of the lower surface. Preferably the top and bottom surfaces are not parallel to one another. Preferably the top and bottom surfaces are inclined relative to one another, ideally in a manner equivalent to a natural disc and/or its nucleus. Preferably the separation of the top and bottom surfaces increases from one side of the core to the other. Preferably the rate of increase in separation is even. Preferably the separation of the top and bottom surfaces increases from the anterior to the posterior side of the core. The top surface and/or bottom surface of the core may be octagonal and/or hexagonal and/or round and/or elliptic. The shape may be regular or irregular, for instance one or more sides of a octagon being larger than one or more others. Rounded corners to the shape are preferably provided.

The core is preferably provided with one or more sides extending between the top surface and the bottom surface. One, six or eight sides may particularly be provided. Where multiple sides are provided, preferably the pairs of opposing sides are provided. The sides may directly or indirectly oppose one another. Preferably opposing sides are parallel to one another, but potentially off set. Preferably the sides are planar. The sides may be vertically provided in use. The anterior side of the core may be of greater width than the posterior side of the core. The anterior side of the core may be of lesser height than the posterior side of the core. The edge of a side closer to the anterior side of the core may be shorter than the edge closer to the posterior side of the core.

The core may be narrower towards the anterior side than towards the middle thereof and/or the core may be narrower towards the posterior side than towards the middle thereof. From anterior to posterior side, the core may have a portion of increasing width, a portion of constant width and a portion of decreasing width.

The core is preferably narrower in the anterior to posterior direction than it is wide, that is perpendicular to the anterior to posterior direction.

The interface between sides of the core and/or between the sides and bottom of the

A particularly preferred form of the core is octagonal in cross-section, increases in thickness from the anterior side to the posterior side and has a shorter anterior side than posterior side. Preferably when viewed in plan, the core lies entirely within the plan of the disc it is to be used to replace.

The core and/or inner component may be provided according to the details of an implant according to the technique set out in applicant's UK Patent Application No 0406851.6 filed 26 March 2004 and/or applicant's UK Patent Application No 0407717.8 filed 5 April 2004.

It is preferred that the anterior edge of the core is recessed relative to the anterior surface of the vertebral bodies and/or anterior edge of the disc prosthesis, preferably by at least 4mm. It is preferred that the centre of the core is provided and maintained at the centre of the disc space. The position of the core relative to the anterior surface of the vertebral bodies and/or anterior edge of the disc prosthesis and/or relative to the centre of the disc space may be maintained by the outer component and/or inner component and/or a spacing component. Preferably the outer component and/or inner component and/or spacing component also thus provide the flanges flush with the anterior surface of the vertebral bodies.

Preferably the position of the core is maintained by a spacing component The spacing component may be a continuation of, and is ideally integral with, the inner component aad/or outer component and/or additional elements. The spacing component is preferably a continuation of one or more of the side walls of the inner component and/or the outer component. Preferably the spacing component is only provided on the anterior side of the core. The spacing component may be formed of folded material. The spacing component may be formed of rolled material. The spacing component may be formed of a pad of material.

Preferably the spacing material is formed by a continuation of the outer component extending across the anterior side of the core, preferably on the outside of the core and/or inside the outer component. The continuation may be doubled back on itself once, twice or more. A further continuation of the outer component may extend across the anterior side of the core, preferably on the outside of the core and/or inside the outer component from the other side of the outer component and/or from the other side relative to the core to the continuation. The further continuation may be doubled back on itself once, twice or more. The continuation and further continuation may have one or more parts provided between one or more parts of the other.

The inner component may be an inner jacket. The inner component may be of fabric.

The fabric may be formed by flat or circular weaving, knitting, braiding, embroidery or combinations thereof.

The fabric may be formed using one or more of polyester, polypropylene, polyethylene, carbon fibre, glass fibre, glass, polyaramide, metal, copolymers, polylactic acid, polyglycolic acid, biodegradable materials, silk, cellulose, silk worm silk, spider silk or polycaprolactone.

Preferably the inner component is separate from the core. Preferably the inner component is separate from the outer component. Relative movement may be facilitated between the inner and outer components. Relative movement between the inner component and core may be allowed. Preferably movement between the inner and outer components is greater than between the inner component and core. Preferably movement between the inner and outer components is facilitated in preference to movement between the inner component and core. Preferably any movement, particularly sliding movement, within the disc is greater between the outer component and inner component than between the inner component and core.

The inner component may entirely surround the core and/or encapsulate the core. One or more apertures or gaps are preferred in the inner component, ideally to provide fluid communication through the inner component. Preferably a large number of apertures or gaps are provided the material from which the inner component is formed, for instance a woven fabric. The apertures or gaps occurring in the inner component due to the manner of manufacture of the material from which it is formed may be supplemented with further apertures or gaps. The supplementation may be provided by degradation and/or absorption of one or more materials forming the inner component.

The inner component may be configured and/or formed of one or more materials intended to promote tissue growth, particularly tissue ingrowth between the inner component and the core and/or through the inner component.

One or more materials used in the inner component may be bio-absorbable and/or soluble and/or degradable, particularly with the spine. The bio-absorbable material may be used to decrease the amount of inner component present and/or positions at which the inner component is present and/or density at which the inner component is present overtime. Areas of bio-absorbable material may be provided. Bio-absorbable fibres may be used to form the inner component. The inner component may be entirely bio-absorbable or only partially. Different materials having different rates of bio-absorption may be used. The may be mixed together in the inner component and/or may be used for particular areas thereof and/or in a particular sequence within the inner component. Slow, moderate and fast bio-absorption materials may be used. Preferably bio-absorption of the inner component is used to provide space for tissue ingrowth.

Preferably the inner component provides a smooth inner surface which potentially contacts the core. Preferably uniform contact between the inner surface of the inner component and the core is provided. Preferably the fibres forming the inner surface of the inner component are evenly positioned with respect to one another. Preferably any abrasion of the core by the inner component is distributed rather than localised. The inner component preferably provides a smooth inner fabric surface, and ideally woven fibrous surface. A densely packed material may be used for the inner surface, ideally to provide the uniform contact surface with the core. The inner surface of the inner component may be of a different material and/or different configuration to the inside and/or outer surface of the inner component.

The inner component may be formed from a substantially planar element. The inner component may be so formed by folding and/or stitching and/or interdigitating one or more parts thereof. In particular, a top wall of the inner component may be connected to a side wall and hence to a bottom wall. One or more further side walls may be connected to the top wall and/or side wall and/or bottom wall. A series of side walls may be provided by an elongate part of the element. Folds or future folds may define one side wall relative to an adjacent side wall or walls.

In a preferred form, the inner component is formed from an element including a side wall connected on one edge to a top wall and connected on an opposing edge to a bottom wall. The respective edges of the side wall are preferably parallel. It is preferred that the side wall will form the side wall at either the anterior, or more preferably, posterior side. Preferably the side wall is connected on one side edge to one or more other side walls, ideally one. Preferably the side wall is connected on the other side edge to one or more other walls, ideally 4 in the case of a hexagonal core and 6 in the case of an octagonal core. The top and bottom edges of the side walls may be parallel or non-parallel depending upon the locations relative to the top and bottom walls they are to occupy. Preferably all the boundaries between side walls in the strip are parallel to one another.

Preferably the side wall(s), top wall and bottom wall are joined together by stitching and/or other attachment techniques.

One or more of the side walls of the inner component may be reinforced and/or of multiple thickness.

On one or more, preferably all, sides, the inner component may be formed of a plurality of inner components. Such a plurality of inner components may be provided in a spiral form or concentric form. Such a plurality of inner components may be integrally formed or may be separately formed. Preferably the plurality of inner components differ from one another in terms of the material from which they are formed and/or the way in which they are formed and/or the properties they provide.

The reinforcement or multiple thickness may be provided by an additional element provided outside of the side wall. The additional element for a side wall may be provided by wrapping one or more additional elements around the side walls. Preferably additional elements are provided for each side wall. Preferably the additional elements are provided by a continuous band extending around the side of the inner component. Preferably the additional elements are configured to substantially match the dimensions of the side wall they contact.

Additional elements may be provided circumferentially around the core and/or inside the outer component. One or more layers of such additional elements may be provided. The one or more layers of additional elements may be free to move relative to one another and/or the core and/or the outer component.

In a preferred form, the additional elements are provided as a continuation of the element providing one or more of the side walls. Preferably the continuation provides 6 or 8 additional elements on the end of the 4 or 6 side walls it already provides.

The additional elements may be joined to the side walls and/or other parts of the inner component by stitching and/or other attachment techniques.

The side walls and/or additional elements may act as an annulus for the disc prosthesis. The side walls and/or additional elements may resists sideways expansion of the core, particularly when under compressive load. The side walls and/or additional elements may provide equivalent properties and/or behaviour to the annulus of a natural disc, for instance during compression and/or distraction and/or horizontal gliding and/or axial rotation and/or flexion and/or extension.

Preferably the core is provided snugly within the inner component. Preferably the top wall and/or bottom wall and/or one or more side walls of the inner component are dimensioned to contact the core.

The outer component may be an outer jacket. The outer component may be of fabric.

The fabric may be formed by flat or circular weaving, knitting, braiding, embroidery or combinations thereof.

The fabric may be formed using one or more of polyester, polypropylene, polyethylene, carbon fibre, glass fibre, glass, polyaramide, metal, copolymers, polylactic acid, polyglycolic acid, biodegradable materials, silk, cellulose, silk worm silk, spider silk or polycaprolactone.

The outer component may entirely surround the inner component and/or encapsulate the inner component. One or more apertures or gaps are preferred in the outer component, ideally to provide fluid communication through the outer component. Preferably a large number of apertures or gaps are provided the material from which the outer component is formed, for instance a woven fabric. The apertures or gaps occurring in the outer component due to the manner of manufacture of the material from which it is formed may be supplemented with further apertures or gaps. The supplementation may be provided by degradation and/or absorption of one or more materials forming the outer component.

The outer component may be configured and/or formed of one or more materials intended to promote tissue growth, particularly tissue ingrowth through the outer component and/or between the inner component and the core and/or through the inner component.

One or more materials used in the outer component may be bio-absorbable and/or soluble and/or degradable, particularly with the spine. The bio-absorbable material may be used to decrease the amount of outer component present and/or positions at which the outer component is present and/or density at which the outer component is present overtime. Areas of bio-absorbable material may be provided. Bio-absorbable fibres may be used to form the outer component. The outer component may be entirely bio-absorbable or only partially. Different materials having different rates of bio-absorption may be used. The may be mixed together in the outer component and/or may be used for particular areas thereof and/or in a particular sequence within the outer component. Slow, moderate and fast bio-absorption materials may be used. Preferably bio-absorption of the outer component is used to provide space for tissue ingrowth.

Preferably the outer component provides a resilient and/or strong containment for the inner component and/or core. Preferably the outer component provides for the anchoring of the prosthesis to the spine.

The outer component may be formed from a substantially planar element. The outer component may be so formed by folding and/or stitching and/or interdigitating one or more parts thereof. In particular, a top wall of the outer component may be connected to a side wall and hence to a bottom wall. One or more further side walls may be connected to the top wall and/or side wall and/or bottom wall. A series of side walls may be provided by an elongate part of the element. Folds or future folds may define one side wall relative to an adjacent side wall or walls.

In a preferred form, the outer component is formed from an element including a side wall connected on one edge to a top wall and connected on an opposing edge to a bottom wall. The respective edges of the side wall are preferably parallel. It is preferred that the side wall will form the side wall at either the anterior, or more preferably, posterior side. Preferably the side wall is connected on one side edge to one or more other side walls, ideally two. Preferably the side wall is connected on the other side edge to one or more other walls, ideally 2 in the case of an octagonal core. A further side wall is preferably connected to the opposite edge of the top wall or bottom wall to the edge to which the side wall linking the top wall and bottom wall is provided. The top and bottom edges of the side walls may be parallel or non-parallel depending upon the locations relative to the top and bottom walls they are to occupy. Preferably all the boundaries between side walls in the strip are parallel to one another.

The outer component may be provided with one or more flanges. Preferably the outer component is provided with at least one flange on one part thereof and at least one other flange on another, preferably opposing, part thereof. Preferably at least one flange which is interdigitated with another, in use, is provided. Preferably one or more edges of the top wall and/or one or more edges of the bottom wall are provided with flanges. Preferably a flange has a length greater than the height of the side walls and/or greater then height of the disc space in which the prosthesis is to be used. The flanges, particularly towards their ends may provide anchor locations for attaching the outer component to one or more vertebrae. Preferably one flange is provided with more anchor locations than another flange, ideally the more anchor locations are provided on the flange for attachment to the inferior and/or lower vertebra. Preferably the one flange is provided with one more anchor locations than the another flange, ideally the more anchor locations are provided on the flange for attachment to the inferior and/or lower vertebra. Preferably the one flange is provided with one anchor location, ideally the more anchor locations are provided on the flange for attachment to the superior and/or upper vertebra. Preferably the another flange is provided with two anchor locations, ideally the more anchor locations are provided on the flange for attachment to the inferior and/or lower vertebra. The anchor locations may be holes, preferably through the flange, and/or fixing receiving locations.

The flanges may have a width less than the width of a side wall. Preferably a first flange has a minimum width less than the minimum width of a second flange, ideally with the one flange having a minimum width less than the minimum width of the another flange. Preferably a first flange has a maximum width less than the maximum width of a second flange, ideally with the one flange having a maximum width less than the maximum width of the another flange. The width of a flange may be considered as the distance from one edge of the flange to another edge in a direction parallel to the disc space and/or perpendicular to the axis of the spinal column and/or across the face of a vertebra, for instance the anterior face. Preferably the first and second flanges, ideally the one flange and the another flange, are of the same length. The length may be considered perpendicular to the width and/or along the axis of the spinal column. Preferably the one flange passes through a hole in the another flange, ideally so as to interdigitated the two flanges.

Preferably a first flange, ideally the one flange, increases in width towards the end of the flange. The first flange, preferably the one flange may taper outward from a reduced neck portion to a wider portion including the anchor location. The wider portion may have a rounded end edge, for instance an edge which has a profile concentric with the fixing. The first flange, ideally the one flange, may be in the form of a finger. Preferably a second flange, ideally the another flange, increases in width towards the end of the flange. The second flange, preferably the another flange may taper outward from a reduced neck portion to a wider portion including the anchor locations. The portion including the anchor locations, particularly a wider portion, may include, at least for a part of the edge, a rounded end edge around each anchor location. The end edge may, in one or more parts, be concentric with a fixing. The portion including the anchor locations, particularly a wider portion, may include a recess in the end edge. The recess may be provided by a part of the flange which is shorted than other parts of the flange, particularly the parts providing the anchor locations. The recess may be provided between the anchor locations and/or part of the flange providing the anchor locations. The recess may be adapted to receive at least a part of the other flange of another disc prosthesis.

The first flange, ideally the one flange, may form a part of the anterior surface profile of the disc prosthesis. Preferably it provides the stem of a Y-shaped profile. Preferably the second flange, ideally the another flange, forms part of the anterior surface profile of the disc prosthesis. Preferably it provides the forks of a Y-shaped profile. Preferably at least a part of the anterior profile of one disc prosthesis, particularly a part of the stem of a Y-shaped profile, may be received between parts of the anterior profile of another disc prosthesis, particularly between the forks of a Y-shaped profile. The at least part of the anterior profile may be so received without any overlap in the material of the one disc prosthesis with the material of the another disc prosthesis.

In a preferred form, a flange is provided on an edge of the top wall which opposes, ideally when considered in the assembled position, an edge of the bottom wall provided with a flange. One of the flanges may be provided with a through aperture. One of the flanges may be provided with a reduced width and/or neck part. Preferably one of the flanges is interdigitated with the other by passing it though the hole. The flange from the top wall is preferably anchored to the bottom vertebrae and the flange from the bottom wall is preferably anchored to the top vertebrae, relative to the disc space being treated, in such a case. One or more pairs of flanges of this type may be provided. The flanges in a pair of flanges may be joined to one another, for instance by a web. The pair of flanges and web may define, at least in part, the boundaries of an aperture.

Preferably the side wall(s), top wall and bottom wall are joined together by stitching and/or other attachment techniques.

The side walls of the outer component may act as an annulus for the disc prosthesis. The side walls of the outer component may resists sideways expansion of the core, particularly when under compressive load. The side walls of the outer component may provide equivalent properties and/or behaviour to the annulus of a natural disc, for instance during compression and/or distraction and/or horizontal gliding and/or axial rotation and/or flexion and/or extension.

Preferably the inner component is provided snugly within the outer component. Preferably the top wall and/or bottom wall and/or one or more side walls of the outer component are dimensioned to contact the inner component.

Preferably the prosthetic disc is anchored to the spine away from the anterior side. Preferably the anchor positions are provided to either side of the anterior of the spine. One or more anchor positions may be used, preferably at least two are used on the vertebrae above and two on the vertebrae below the disc being replaced.

Preferably the prosthetic disc is anchored to the spine using one or more anchor locations provided thereon. Preferably one or more anchor locations are provided by a flange or flanges provided by the outer component. Preferably a flange has a length greater than the height of the side walls and/or greater then height of the disc space in which the prosthesis is to be used. The flanges may provided the anchor locations towards their ends. The flanges may have a width less than the width of a side wall.

In a preferred form, a flange is provided on the outer component in opposition to another flange provided on another part of the outer component. One of the flanges may be provided with a through aperture. One of the flanges may be provided with a reduced width and/or neck part. Preferably one of the flanges is interdigitated with the other by passing it though the hole. The flange from the top wall is preferably anchored to the bottom vertebrae and the flange from the bottom wall is preferably anchored to the top vertebrae, relative to the disc space being treated, in such a case. One or more pairs of flanges of this type may be provided.

The outer component may be fastened at the anchor positions to one or more adjacent vertebra, for instance using fasteners. The fasteners may be one or more of bone screws, staples, sutures, nails or the like.

Where no inner component is provided, the preferably the outer component is separate from the core. Relative movement between the outer component and core may be allowed. The outer component may entirely surround the core and/or encapsulate the core. One or more apertures or gaps are preferred in the outer component, ideally to provide fluid communication through the outer component. Preferably a large number of apertures or gaps are provided the material from which the outer component is formed, for instance a woven fabric. The apertures or gaps occurring in the outer component due to the manner of manufacture of the material from which it is formed may be supplemented with further apertures or gaps. The supplementation may be provided by degradation and/or absorption of one or more materials forming the outer component. Preferably the outer component provides a smooth inner surface which potentially contacts the core. Preferably uniform contact between the inner surface of the outer component and the core is provided. Preferably the fibres forming the inner surface of the outer component are evenly positioned with respect to one another. Preferably any abrasion of the core by the outer component is distributed rather than localised. The outer component preferably provides a smooth inner fabric surface, and ideally woven fibrous surface. A densely packed material may be used for the inner surface, ideally to provide the uniform contact surface with the core. The inner surface of the outer component may be of a different material and/or different configuration to the inside and/or outer surface of the outer component.

The disc prosthesis may include absorbable, for instance bio-absorbable, material between the anchor position or positions of the prosthesis and the outer component of the prosthesis. The disc prosthesis may include absorbable, for instance bio-absorbable, material between a part of the flange or flanges of the prosthesis and the outer component of the prosthesis.

The anchor position(s) and/or at least a part of the flange(s) may be joined to the disc prosthesis, particularly the outer component thereof, by an absorbable zone. The absorbable zone may be formed entirely of absorbable material. The absorbable material may be made of fibres. The absorbable zone may provide the only joint with the disc prosthesis, particularly the outer component thereof. The absorbable zone may make the anchor position(s) and/or at least a part of the flange(s) detachable from the disc prosthesis, particularly the outer component thereof.

The anchor position(s) and/or more particularly at least a part of the flange(s) may be formed from at least two different materials. At least one absorbable material is preferably provided. At least one non-absorbable material is preferably provided. Preferably at least one of the materials is used to provide the load bearing function, preferably the load bearing fibres. Preferably the load bearing material is made of absorbable material, particularly absorbable fibres. Preferably the at least one non-absorbable material defines the overall shape of the flange(s) and/or maintain the interdigitation of flanges and/or is subjected to level of tension, particularly after absorption of the absorbable material. The absorbable material may surround the non-absorbable material.

The anchor position(s) and/or at least a part of the flange(s) may be joined to the disc prosthesis, particularly the outer component, by a plurality of different material, particularly fibre, configurations and/or types. A material having a non-linear configuration, particularly in terms of the fibres forming it may be provided. The non-linear material and/or fibres may be curved and/or spiralled and/or serpentine and/or zigzag in configuration. The non-linear material and/or fibres may have a first form and a second form. In the second form, the length of the material and/or fibres being greater in the second form and/or the material and/or fibres may be more linear. Preferably the non-linear material and/or fibres are not load bearing at the first time and/or at implantation and/or in the first form. The non-linear material and/or fibres may be maintained in the first form by a further material and/or further fibres. The further material and/or fibres may be absorbable. Preferably the further material and/or further fibres are load bearing at the first time and/or at implantations and/or in their first form. Preferably the further material and/or fibres are present in their first form and absent, preferably due to absorption, in their second form. The non-linear material and further material may be separate from one another. The further material may surround the non-linear material, for instance as a sleeve. The further material may be mixed or intermingled with the non-linear material. The further material may isolate the non-linear material from the load in the first form. The further material may be attached to the non-linear material in the first form. The attachment may be through adhesion to and/or winding round and/or stitching to the further material. The further material may act as a bridging material between parts of the non-linear material.

The absorbable material may be provided in one or more forms. A plurality of forms may be provided. The plurality of forms may provide for different rates of absorption. The different forms may different in terms of one or more of their material and/or diameters and/or dimensions and/or densities and/or bulk densities. The absorbable materials and/or non-absorbable materials may be provided in one or more in-growth controlling forms. Different in-growth controlling forms may be used to give different extents of tissue ingrowth with time. Different in-growth controlling forms may be used to give different in growth extents for different parts of the prosthesis, and particularly within different parts of the flanges. The different extents may be between zero and the maximum possible.

The anchor position(s) and/or the flange(s) may be provided with suture receiving sections. The suture receiving sections may be provided on all flanges and preferably define the anchor positions. The suture receiving sections may include one or more suture bearing parts. The suture bearing parts may be reinforced part, for instance one or more reinforced bands. One of more of the suture receiving parts may extend across the flange and/or perpendicular to the direction of load and/or tension. One or more of the suture receiving parts may extend across the flanges between fibres, particularly load bearing fibres, on one side of the flange and fibres, particularly load bearing fibres, on the other side of the flange. A series of suture receiving sections are preferably provided, preferably spaced along the length of the flanges. Between the suture bearing parts, one or more openings may be provided. Preferably one or more of the openings are spanned by one of more fibres, and ideally by a mesh. Preferably a suture is passed through the opening, round the suture bearing part and through an opening on the other side of the suture bearing part. Preferably multiple loops of the suture are provided. Preferably a plurality of anchor positions are provided along the length of the flange(s). Preferably a plurality of suture receiving sections and/or suture bearing parts are provided along the length of the flange.

The invention may include any of the features, options or possibilities set out elsewhere in this document.

Also described below is a kit for use in providing a disc prosthesis, the kit including a series of different sized prostheses, one or more of the prostheses including a core, the core preferably being provided within an inner component, the inner component being provided within an outer component or the core being provided with an outer component.

Preferably the kit includes Different sized prostheses for different sized patients and/or different sized prostheses sized for different discs of the spine and particularly the lumber region thereof.

The kit may include any of the features, options or possibilities set out elsewhere in this document.

Also described below is a surgical technique for providing a disc prosthesis, the technique including, removing at least patt of the natural disc in a spine and inserting a disc prosthesis in the spine, the disc prosthesis composing a core.

Preferably the core is provided within an inner component or within an outer component. Preferably the inner component is provided within an outer component.

The technique may be performed anterially or posterially.

The technique may use a pre-assembled prosthesis. Preferably the outer component is inserted into the space and the inner component and core are then inserted. The inner component and core may be provided pre-assembled. A plurality of cores may be inserted into a single outer component.

The method may include forming the core in situ. For instance, multiple components may be used to form the core. The method may be a minimally invasive surgical technique, particularly where the core is formed in the inner component in-situ. The inner component may be inserted and then filled with the core. The outer component may be inserted then have the inner component provided within it, potentially then being filled with core.

The core material and/or inner component may particularly be formed in-situ according to the technique set out in applicant's UK Patent Application No 0406851.6 filed 26 March 2004 and/or UK Patent Application No 0407717.8 filed 5 April 2004.

Preferably the level of tension and/or load between the anchor position or positions of the disc prosthesis and the outer component of the disc prosthesis vary between a first time and a second time. The first time may be the time of implantation, for instance 1 hour after implantation, or perhaps 1 day after implantation. The second time may be a time after implantation, for instance at least 30 days, preferably at least 60 days, more preferably at least 100 days and potentially even at least 300 days, after implantation. Preferably the level of tension and/or load is lower at the second time than at the first time. Preferably the level of tension and/or load is lower after biological in-growth has occurred. The ingrowth may be into the outer component and/or inner component and/or flanges. Preferably the range of extension of the spine at the first time is less than the range of extension at the second time. Preferably the transition between the level of load and/or level of tension and/or range of extension at the first time and at the second time is phased or gradual. The transition may occur evenly through out the time between the first time and the second time, but preferably occurs during a time period starting after the first time. The transition may continue after the second time to a still lower level of tension and/or load and/or to a still higher range of extension.

The method may include using a disc prosthesis provided with at least one flange on one part thereof and at least one other flange on another, preferably opposing, part thereof. Preferably the method includes at least one flange being interdigitated with another flange, preferably by passing the one flange through a hole in the another flange. The method may include introducing one or more fixings to anchor locations, preferably provided towards the ends of the flange(s). Preferably the method includes providing one flange with more fixings than another flange, ideally the more fixings are provided on the flange for attachment to the inferior and/or lower vertebra. Preferably the method includes provided one flange with one more fixing than the another flange, ideally the more fixings are provided on the flange for attachment to the inferior and/or lower vertebra. Preferably the method includes providing the one flange with one fixing, ideally the one fixing is provided on the flange for attachment to the superior and/or upper vertebra, and providing the another flange with two fixings, ideally the two fixings are provided on the flange for attachment to the inferior and/or lower vertebra.

The method may include using a flange provided with a recess, particularly in the end thereof. The end may be that part of the flange furthest from the core. The method may include providing fixings through the flange to either side of the recess: The method may include providing a further disc prosthesis, preferably of the same type, for an adjacent disc space to that the disc prosthesis is provided in. The method may include fixing a flange of the disc prosthesis and a flange of the further disc prosthesis to the same vertebra. The method may include a providing at least a part of one disc prosthesis between at least a part of another disc prosthesis. The part may be provided within the recess. The part may be provided within a recess provided between the anchor locations and/or part of the flange providing the anchor locations and/or the fixings.

The method may include the use of a first flange, ideally the one flange, to form a part of the anterior surface profile of the disc prosthesis. Preferably the method includes the provision as a part of the profile of the stem of a Y-shaped profile. Preferably the method includes the use of a second flange, ideally the another flange, to form part of the anterior surface profile of the disc prosthesis. Preferably the method includes the provision as a part of the profile of the forks of a Y-shaped profile. Preferably at least a part of the anterior profile of one disc prosthesis, particularly a part of the stem of a Y-shaped profile, is provided between parts of the anterior profile of another disc prosthesis, particularly between the forks of a Y-shaped profile, as a part of the method. The method preferably includes the at least part of the anterior profile being so provided without any overlap in the material of the one disc prosthesis with the material of the another disc prosthesis.

The method may include any of the features, options or possibilities set out elsewhere in this document.

Also described below is a disc prosthesis, the disc prosthesis including an outer component, the outer component being provided with at least one flange on one part thereof and at least one other flange on another part thereof.

Preferably at least one flange which is interdigitated with another, in use, is provided. Preferably one or more edges of the top wall and/or one or more edges of the bottom wall are provided with flanges. Preferably a flange has a length greater than the height of the side walls and/or greater then height of the disc space in which the prosthesis is to be used. The flanges, particularly towards their ends may provide anchor locations for attaching the outer component to one or more vertebrae. Preferably one flange is provided with more anchor locations than another flange, ideally the more anchor locations are provided on the flange for attachment to the inferior and/or lower vertebra. Preferably the one flange is provided with one more anchor locations than the another flange, ideally the more anchor locations are provided on the flange for attachment to the inferior and/or lower vertebra. Preferably the one flange is provided with one anchor location, ideally the one anchor location is provided on the flange for attachment to the superior and/or upper vertebra and the another flange is provided with two anchor locations, ideally the two anchor locations are provided on the flange for attachment to the inferior and/or lower vertebra. The anchor locations may be holes, preferably through the flange, and/or fixing receiving locations.

The flanges may have a width less than the width of a side wall. Preferably a first flange has a minimum width less than the minimum width of a second flange, ideally with the one flange having a minimum width less than the minimum width of the another flange. Preferably a first flange has a maximum width less than the maximum width of a second flange, ideally with the one flange having a maximum width less than the maximum width of the another flange. The width of a flange may be considered as the distance from one edge of the flange to another edge in a direction parallel to the disc space and/or perpendicular to the axis of the spinal column and/or across the face of a vertebra, for instance the anterior face. Preferably the first and second flanges, ideally the one flange and the another flange, are of the same length. The length may be considered perpendicular to the width and/or along the axis of the spinal column. Preferably the one flange passes through a hole in the another flange, ideally so as to interdigitated the two flanges.

Preferably a first flange, ideally the one flange, increases in width towards the end of the flange. The first flange, preferably the one flange may taper outward from a reduced neck portion to a wider portion including the anchor location. The wider portion may have a rounded end edge, for instance an edge which has a profile concentric with the fixing. The first flange, ideally the one flange, may be in the form of a finger. Preferably a second flange, ideally the another flange, increases in width towards the end of the flange. The second flange, preferably the another flange may taper outward from a reduced neck portion to a wider portion including the anchor locations. The portion including the anchor locations, particularly a wider portion, may include, at least for a part of the edge, a rounded end edge around each anchor location. The end edge may, in one or more parts, be concentric with a fixing. The portion including the anchor locations, particularly a wider portion, may include a recess in the end edge. The recess may be provided by a part of the flange which is shorted than other parts of the flange, particularly the parts providing the anchor locations. The recess may be provided between the anchor locations and/or part of the flange providing the anchor locations. The recess may be adapted to receive at least a part of the other flange of another disc prosthesis.

The first flange, ideally the one flange, may form a part of the anterior surface profile of the disc prosthesis. Preferably it provides the stem of a Y-shaped profile. Preferably the second flange, ideally the another flange, forms part of the anterior surface profile of the disc prosthesis. Preferably it provides the forks of a Y-shaped profile. Preferably at least a part of the anterior profile of one disc prosthesis, particularly a part of the stem of a Y-shaped profile, may be received between parts of the anterior profile of another disc prosthesis, particularly between the forks of a Y-shaped profile. The at least part of the anterior profile may be so received without any overlap in the material of the one disc prosthesis with the material of the another disc prosthesis.

The prosthesis may include any of the features, options or possibilities set out elsewhere in this document.

Also described below is a surgical technique for providing a disc prosthesis, the technique including, removing at least part of the natural disc in a spine and inserting a disc prosthesis in the spine, the disc prosthesis including an outer component, the outer component being provided with at least one flange on one part thereof and at least one other flange on another part thereof

The method may include using a disc prosthesis provided with at least one flange on one part thereof and at least one other flange on another, preferably, opposing, part thereof. Preferably the method includes at least one flange being interdigitated with another flange, preferably by passing the one flange through a hole in the another flange. The method may include introducing one or more fixings to anchor locations, preferably provided towards the ends of the flange(s). Preferably the method includes providing one flange with more fixings than another flange, ideally the more fixings are provided on the flange for attachment to the inferior and/or lower vertebra. Preferably the method includes provided one flange with one more fixing than the another flange, ideally the more fixings provided on the flange for attachment to the inferior and/or lower vertebra. Preferably the method includes providing the one flange with one fixing, ideally the one fixing is provided on the flange for attachment to the inferior and/or lower vertebra and providing the another flange with two fixings, ideally the two fixings are provided on the flange for attachment to the inferior and/or lower vertebra.

The method may include using a flange provided with a recess, particularly in the end thereof. The end may be that part of the flange furthest from the core. The method may include providing fixings through the flange to either side of the recess. The method may include providing a further disc prosthesis, preferably of the same type, for an adjacent disc space to that the disc prosthesis is provided in. The method may include fixing a flange of the disc prosthesis and a flange of the further disc prosthesis to the same vertebra. The method may include a providing at least a part of one disc prosthesis between at least a part of another disc prosthesis. The part may be provided within the recess. The part may be provided within a recess provided between the anchor locations and/or part of the flange providing the anchor locations and/or the fixings.

The method may include the use of a first flange, ideally the one flange, to form a part of the anterior surface profile of the disc prosthesis. Preferably the method includes the provision as a part of the profile of the stem of a Y-shaped profile. Preferably the method includes the use of a second flange, ideally the another flange, to form part of the anterior surface profile of the disc prosthesis. Preferably the method includes the provision as a part of the profile of the forks of a Y-shaped profile. Preferably at least a part of the anterior profile of one disc prosthesis, particularly a part of the stem of a Y-shaped profile, is provided between parts of the anterior profile of another disc prostheses, particularly between the forks of a Y-shaped profile, as a part of the method. The method preferably includes the at least part of the anterior profile being so provided without any overlap in the material of the one disc prosthesis with the material of the another disc prosthesis.

The surgical technique may include any of the features, options or possibilities set out elsewhere in this document.

Various embodiments of the invention will now be described, by way of example only, and with reference to the accompanying drawings in which:-
Figure 1 is a plan view of a core suitable for use in the present invention;
Figure 2 is a cross-sectional front view of the core of Figure 1;
Figure 3 is a cross-sectional side view of the core of Figure 1;
Figure 4 is a plan view comparing the profile of a core according to the invention with a natural disc;
Figure 5 illustrates an inner jacket according to the present invention, prior to assembly;
Figure 6 illustrates an outer jacket according to the present invention, prior to assembly;
Figure 7 illustrates an outer jacket according to another embodiment of the present invention, prior to assembly;
Figure 8a, 8b and 8c show respectively an assembled disc outer, disc outer in plan view and disc outer in combination with core;
Figure 9a, 9b and 9c show respectively an assembled disc outer with an inner, annular reinforcement, the disc outer in plan view and the disc outer in plan view with the inner annular reinforcement and core;
Figure 10a and 10b show respectively an assembled disc outer with inner reinforcement and core and plan view of the same;
Figure 11a illustrates a further embodiment of the outer jacket prior to assembly;
Figure 11b illustrates the embodiment of Figure 11a in assembled format in a plan view;
Figure 11c illustrates the embodiment of Figure 11a in assembled, perspective view;
Figure 12a illustrates a view of an embodiment of an inner reinforcement, prior to assembly;
Figure 12b illustrates the outer of Figure 12a in assembled form, in plan view; Figure 12c shows the inner of Figure 12a in assembled form, and contained within an outer jacket;
Figure 13 shows a still further embodiment of an outer jacket, prior to assembly;
Figure 14a shows an embodiment of a disc outer potentially assembled from a disc outer according to Figure 13;
Figure 14b shows an assembled disc outer with buttress elements, potentially formed from an outer jacket according to Figure 13;
Figure 14c shows an assembled disc outer with buttress elements, potentially formed from an outer jacket according to Figure 13;
Figure 14d is a perspective view of an assembled outer jacket including the buttress elements;
Figure 15a shows another embodiment of an outer jacket, prior to assembly; Figure 15b shows the embodiment of Figure 15a, with certain sections highlighted;
Figure 16 illustrates an assembled outer jacket according to one form, left hand side, and according to another form, right hand side;
Figure 17 illustrates the use of two assembled discs, with outer jackets according to the another form of Figure 16, between adjacent vertebrae and
Figure 18 illustrates in a closer view the use of two assembled discs, with outer jackets according to the another form of Figure 16, between adjacent vertebrae.

The prior art contains examples of elastomeric discs, with the motion of the elastomer being contained by bonding it to metallic end-plates. In use, this results in high strains at the exterior faces of the disc and this in turn can give rise to tearing and eventually failure of the core.

The previously developed artificial intervertebral disc detailed in US-6093205, was developed particularly for the cervical region of the spine. The combination of an elastomeric inner core surrounded by a single embroidered outer textiles jacket has been shown to offer particular benefit in terms of the encapsulation preventing the initiation or propagation of any fissures in the elastomer component of the artificial disc.

To provide an optimised artificial disc for use in the lumbar region of the spine a number of further developments and improvements have been made. The artificial disc may act as a complete disc replacement, or a partial replacement, for instance for the nucleus. Anterior or posterior insertion is possible. The further developments and improvements are also useful in the context of other disc prostheses too.

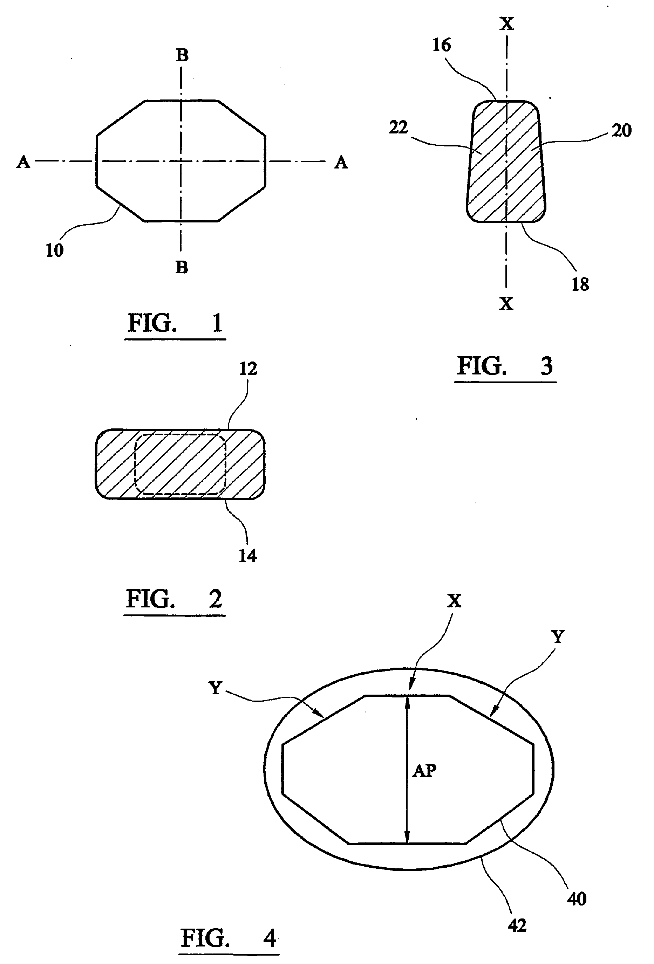

Firstly the core design has been designed specifically to provide optimal performance in the context of the lumbar region. Figure 1 represents a plan view of the core, in effect looking down on the disc as positioned in the spine, with the anterior top and posterior bottom in the figure. The core is octagonal with a greater width (left to right in the figure) than depth (top to bottom). The sides 10 are planar with rounded corners between them. The core is made of a long term implantable grade silicone material. A 50° Shore hardness material is preferred. Figure 2 is a cross-sectional view along axis A-A of Figure 1 and hence is a view of the posterior half of the core viewed from the anterior side. The planar upper surface 12 and lower surface 14 are visible: Figure 3 is a cross-sectional view along axis B-B of Figure 1 and hence shows the transition from anterior to posterior side. As can be seen, the thickness at the anterior edge 16 is less than the thickness at the posterior edge 18. Both upper 20 and lower 22 sides of the core increase symmetrically in thickness relative to the centreline of the core X-X during the transition from anterior edge 16 to posterior edge 18.

The optimised core design's plan profile 40 is seen in comparison with the plan profile 42 of the natural disc it is intended to replace in Figure 4. The naturally curved shape of the disc has been squared off in to an octagonal design. This allows easier design of the embroidery element of the disc. Additionally the anterior to posterior length, AP dimension, is reduced compared with the natural disc so as to keep the artificial disc away from the great vessels. When anchoring the device, as described in more detail below, centrally located anchoring on the anterior face, position X, of the vertebrae is avoided, with a preference for anchoring on the adjacent sides, positions Y.

Various alternative constructions of the core around this basic principle can be used. The core could be constructed as a single piece, in a manner such as that suggested above. Alternatively, particularly where minimally invasive surgery is required, the core may be formed of multiple core pieces which are inserted and assembled to form the overall core in-situ. Such core pieces can be individually inserted and assembled within a single inner jacket, but more preferably are individually wrapped in inner jackets which are then maintained in position by a single outer jacket.

In more varied forms, the core can be formed of potentially tens or hundreds of small beads. The inner jacket would serve to maintain these in position. Cores formed of elastomer or hydrogel with elastomeric properties are also possible. As alternatives to the illustrated octagonal shape, hexagonal or rounded shapes can be used.

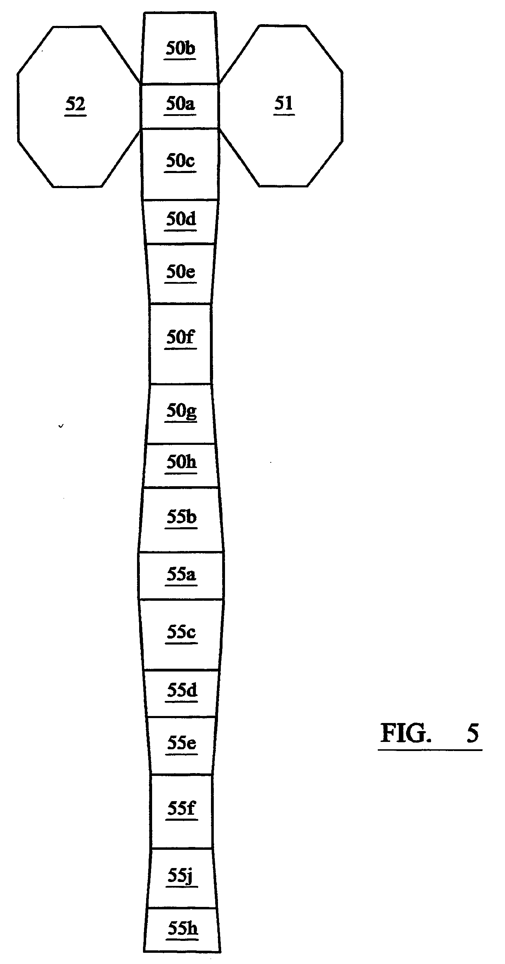

Around the core, an inner jacket is provided. This may be embroidered and/or woven. This is separate from a subsequent outer jacket. The inner jacket provides complete encapsulation of the core. As shown in Figure 5, the jacket is in the form of a first side wall 50a which is connected to a top wall 51 and bottom wall 52. The first side wall 50a is connected to a second side wall 50b in a first direction. In a second direction, the first side wall 50a is connected, in sequence to a third side wall 50c, fourth side wall 50d, fifth side wall 50e, sixth side wall 50f, seventh 50g and eighth 50h. These side wall are stitched to the top wall 51 and bottom wall 52 so as to give an octagonal box form to the inner jacket and close completely around the core.

The material used for the inner jacket uses densely packed fibres to define as smooth a surface as possible for the fabric. This is particularly desirable for the inner surfaces which contact the core. This ensures the most uniform contact surface area between the inner jacket and the elastomer core.

Connected to the eighth side wall 50h is the first of a series of additional elements also formed from the same embroidery. These additional elements, in sequence 55b, 55a, 55c, 55d, 55e, 55f, 55g and 55h are wrapped around the side walls 50 of the assembled inner jacket. As a result they form an additional ring of material around the side of the core. In effect this extra band of material strengthens the ability of the inner jacket to act as a natural annulus would and resist expansion sideways by the core when placed under compressive load. The additional elements can be secured with further stitching. The additional elements 55 could of course be provided by a suitably configured, but separate element to the element providing the walls 51, 52, 50.

The side walls 50 and additional elements 55 are provided with a length and height pattern intended to define an inner jacket which matches the length and height variation pattern of the core.

An inner jacket provided in this way offers at least two key benefits.

Firstly it allows the jacket in contact with the core to have relatively low movement levels, whilst still enabling the overall desired level of movement for the artificial disc due to the outer jacket's presence and design. Low movement levels for the inner jacket mean that abrasion of the core is minimised. A single jacket would not achieve this.

Secondly, the inner jacket can be designed with properties ideal for its purpose, whilst allowing the outer jacket to be designed with properties ideal for its purpose. Thus the inner jacket aims to provide as dense and hence smooth a fabric surface as possible in contact with the core. In this way the risk of individual fibres protruding relative to the others is reduced. Protruding fibres can potentially cause wear due to the micro-motion of the jacket against the core in use. This is a particular potential issue in the context of the high loads encountered in the lumber region. Whilst such properties are desirable here, they are not consistent with those found to be desirable for the outer surface/outer jacket of the artificial disc. Using two separate jackets allows better optimisation in each case.

In a modified embodiment of the inner jacket, its properties may be tailored to facilitate tissue ingrowth into the space between the inner jacket and the core. The formation of a layer of tissue directly between the jacket and the core of the disc should be beneficial in reducing still further wear in the device. Because the dense fibre form used to provide the most smooth surface contacting the core is not the most conducive to tissue ingrowth, the make up of the inner jacket may be carefully controlled to assist.

By forming the inner jacket with a portion of the fibres or material formed of bio-absorbable material, as tissue ingrowth occurs the inner jacket can be partially absorbed to provide further room for the ingrowth. The non-bioabsorbable material of the inner jacket serves to provide the required structure for the inner jacket over its lifetime, supplemented by the assistance provided by the tissue itself. The use of quickly, moderately and slowly absorbed biomaterials in conjunction with non-absorbable materials can provide a gradual transition from the desired function being provided by the inner jacket alone to the point where it is shared between jacket and tissue. In some cases, an entirely bio-absorbable inner jacket may be provided. Various distributions for the non-absorbable and bio-absorbable material are possible in the inner jacket. The non-absorbable material may particularly form the outside of the inner jacket.

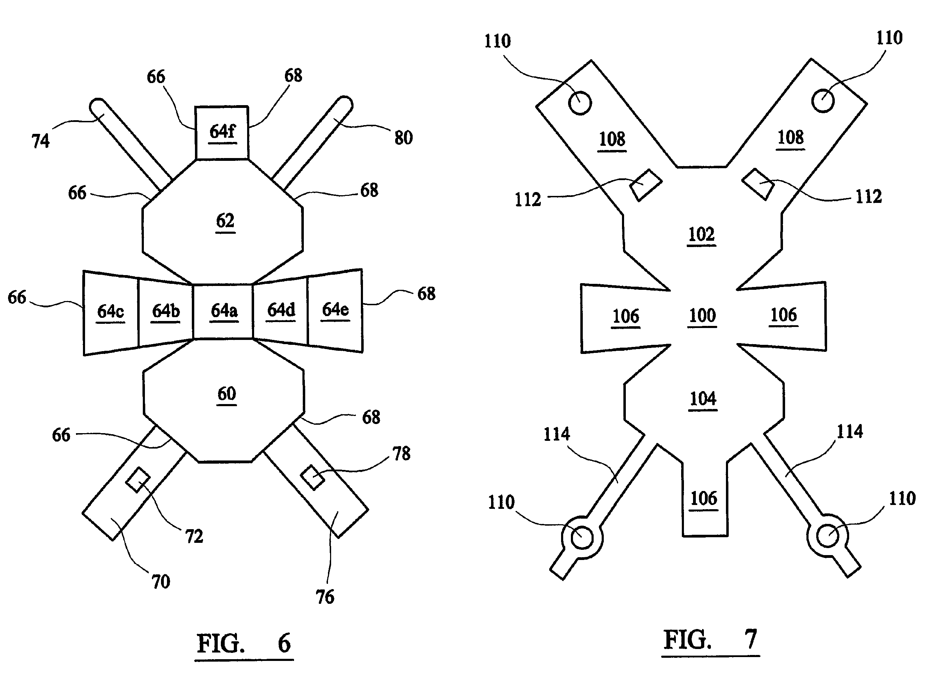

In addition to the core and inner jacket, an outer jacket is provided. A suitable outer jacket is illustrated in Figure 6. This is intended to substantially surround the inner jacket. The outer jacket has a bottom wall 60 and top wall 62, which are connected by side wall 64a. Further side walls 64b 64c are provided to one side of side wall 64a. Further side walls 64d, 64e are provided to the other side of side wall 64a. Attached to the top wall 62 is a sixth side wall 64f. The top, bottom and side walls are connected to one another by stitching. This leaves two sides of the outer jacket open, in effect the openings defined by edges 66 in one case and 68 in the other.

The edge 66 of the bottom wall 60 is provided with a flange 70. This has a hole 72 in it. The edge 66 of the top wall 62 is provided with a flange 74 which is thinner than flange 70, so as to be able to pass through the hole 72 in flange 70. Similarly, the edge 68 of the bottom wall 60 is provided with a flange 76. This has a hole 78 in it. The edge 68 of the top wall 62 is provided with a flange 80 which is thinner than flange 76, so as to be able to pass through the hole 78 in flange 76. To close the remaining two sides, therefore, flanges 70 and 74 and flanges 76 and 80 are interdigitated.

The flanges 70, 74, 76 and 80 are all significantly longer than the height of the disc space the artificial disc is to be used in. As a result the ends 82 of the flanges 70, 74, 76, 80 can be anchored to the vertebra above the disc replacement in the case of flanges 70 and 76 and to the vertebra below the disc replacement in the case of the flanges 74, 80.

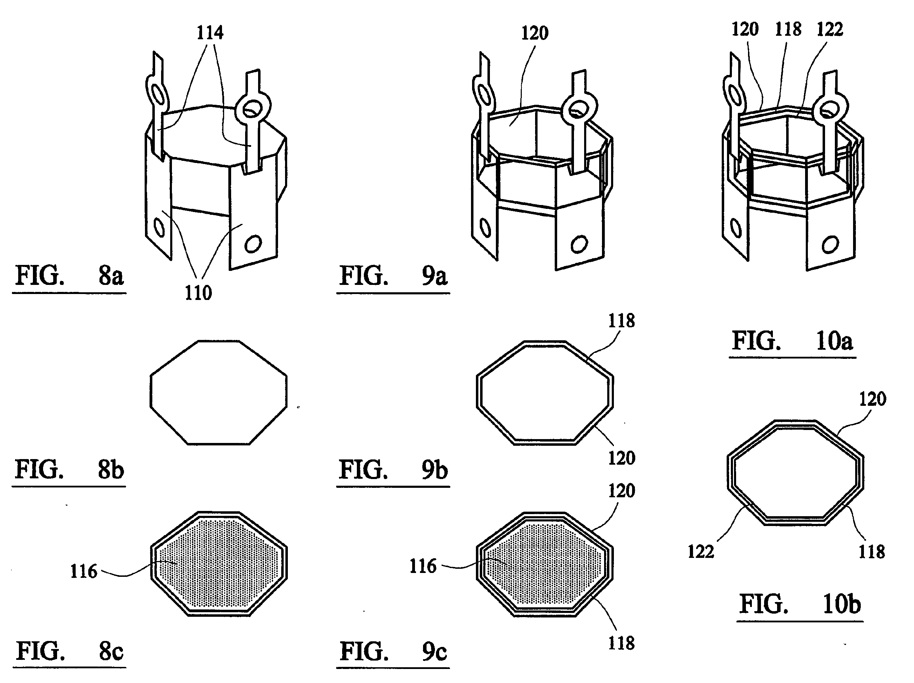

A similar outer jacket to that illustrated in Figure 6 is provided in Figure 7. In this case, bottom wall 100 is connected to the top wall 102 by means of side wall 104. Further side walls 106 are provided. Two flanges 108 are provided connected to the top wall 102. These flanges are provided with a hole 110 in each case which is intended to receive the fixing used to collect the device to the spine. These holes are provided towards the ends of the flanges. Close to the top wall 102 two further holes 112 are provided. These have the inner flanges 114 which are connected to the bottom wall 100 passed through them in use, see Figure 8a. These flanges are also provided with holes 110 to receive fixings in use.

In its assembled form, such a disc outer can appear as shown in Figure 8a. Here the flanges 114 are clearly shown as interdigitated with the flanges 110 by virtue of their being passed through the holes 112 therein. The completed structure formed by the bottom wall 100, top wall 102, side wall 104 and further side walls 106, together with the flanges, totally encloses the core. Once again, an octagonal plan view is provided, Figure 8b, with a similarly shaped octagonal core 116 provided therein, Figure 8c. The core 116 in this case, as with the previous embodiments, is generally centred within the outer jacket.

In the Figure 9a, 9b and 9c embodiment, an additional ring of material is provided around the core, inside the outer jacket 118 by an inner 120. In practice, this provides additional strength to the device when resisting lateral expansion when the core is compressed, i.e. into or out of the paper in the plan view shown in Figure 9c.

The Figure 9a embodiment shows in perspective view the overall assembly consisting of the outer jacket, inner reinforcement and core. In this case an additional annular reinforcement 122 is provided.

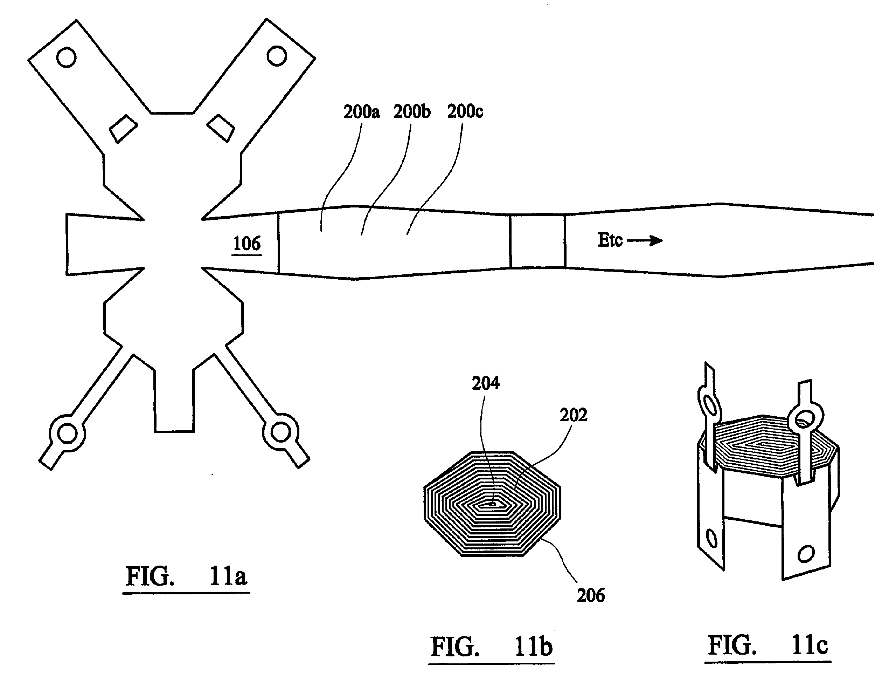

The Figure 11a embodiment of the invention provides for a similar outer jacket to that described in Figure 7 above. However, in this case, the side walls 106 are extended by a very substantial amount via a series of additional elements 200a, 200b, 200c etc. A large number of repeats of these additional elements are provided, a number too great to be shown on the Figure 11a drawing sheet. This device is assembled by folding the additional elements, starting at one end, so as to form a spiral of generally octagonal outline. The result is shown in Figure 11b where a spiral 202 is formed extending from the very centre of the device 204, out to its outer wall 206. Such a spiral can provide the core itself, or additional core material can be provided between the turns of the spiral, for instance hydrogel or other material which can be caused to flow into the device and then allowed to set. In Figure 11c, an interdigitated, assembled form of the device of Figure 11a and Figure 11b is shown. The spiral core forms the core function for this device, as well as providing substantial reinforcement against expansion when the device is placed under compression. In effect the spiral provides the core, inner component and outer component in this embodiment.

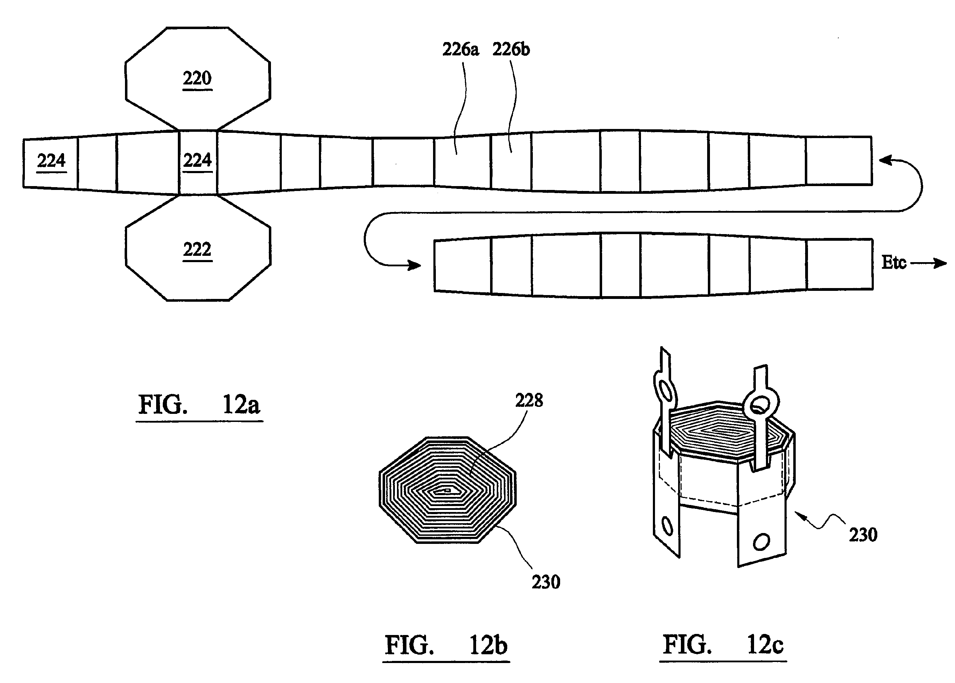

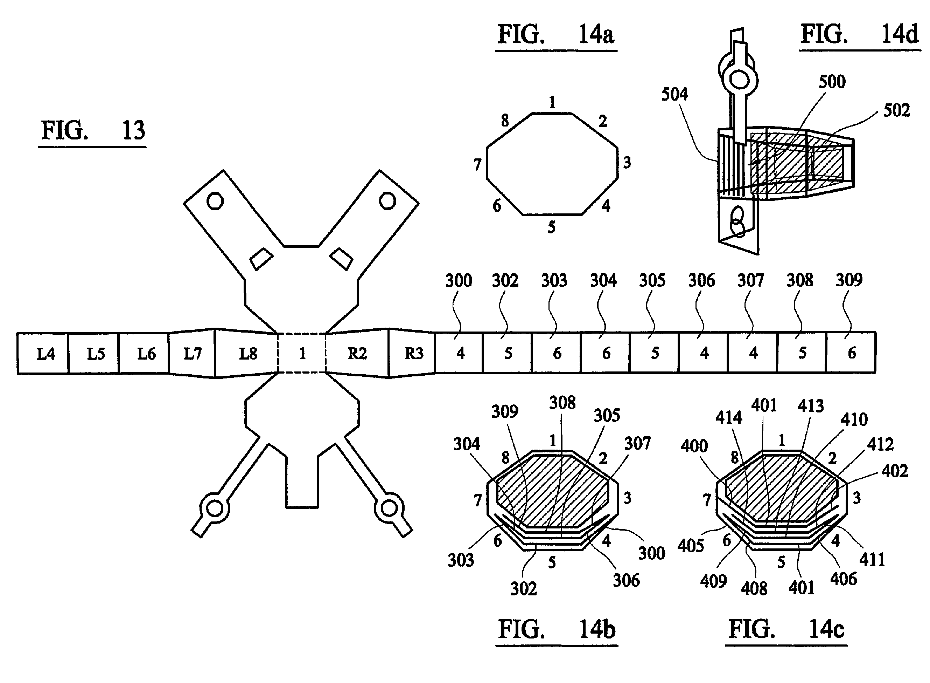

In Figure 12a, an unassembled form for the inner component is provided, including top wall 220, bottom wall 222, side walls 224 and a large number of additional elements 226a, 226b etc. Once again, these additional elements can be folded so as to provide an octagonal spiral core with the walls 224, 220 and 222 completing the exterior 228 of this inner component. This in turn is received within an outer component 230, the assembled form for which is shown in Figure 12c. Again, the folded additional elements may form the core on their own or together with other core material, such as hydrogels. Again, a core structure of this type provides substantial resistance to sideways expansion when the device is placed under compression. In the Figure 13 and Figure 14a to 14d illustrations, a form of device is provided in which the centre of the core is correctly located in the centre of the disc space it is to be provided in. This is achieved by the use of a buttress zone formed in the device. This structure for the device allows the fixation flanges, with their interdigitation, to be flush with the anterior surface of the vertebral bodies, but still allow the disc itself to sit recessed by at least 4mm within the disc space. Correct centring of the core, acting as the replacement, is thus provided. Additionally, such replacement reduces the risk of the main body of the device being pinched by the anterior lip of the vertebrae as the spine is flexed.

Whilst it possible to form the buttress from an entirely separate component, such as a folded fabric, in the preferred format, it is formed from a series of further elements 300 through to 309. In effect, side walls are provided on the left hand side of the device, as seen in the simple plan view in Figure 14a by means of the panel L8, L7, L6 and L4. The right hand side is provided by panels R2, R3. The further elements 300 through to 309 are folded to form the buttress structure. A variety of configurations are possible, but in the illustrated form of Figure 14b, the first part of the buttress is formed by panel 300 which extends inside the outer profile of outer jacket from the edge formed by the contact of panel R3 and L4. Further element 302 extends across the end of panel L5, further element 303 across the inside of panel L6. The further element 304 is then folded back across the inside of further element 303, with further element 305 being across the inside of further element 302. Similarly, further element 306 is provided across the inside of further element 300, before there is a further fold so as to provide further element 307 across the inside of further element 306. Further element 308 is provided across the inside of further element 305 with further element 309 being provided across the inside of the further element 304. Further folds of material can be provided if needed.

An alternative format for the buttress structure, formed in a similar way, is shown in Figure 14c. Here, further elements provided at one end of the outer jacket form the inner most further elements 400, 401 and 402. Further elements provided between there and the outer wall 405 of the outer jacket are provided by further element 406 through to 414, with further element 414 being the end and lying between further element 400 and further element 409.

A perspective view of such a device, showing the anterior edge 500 of the core 502 recessed relative to the anterior edge 504 of the overall device is shown in Figure 14d.

The outer jacket has at least three beneficial functions.

Firstly, it provides a jacket against the vertebral end-plates which is separate from the inner jacket that surrounds the core. This reduces micro-motion between the core and the inner jacket, but still means that the overall level of movement is as desired for the disc replacement as a whole.

Secondly, the outer jacket serves to effectively anchor the artificial disc in place. The interdigitation of the outer jacket effectively retains the inner jacket and core within it. Furthermore, the anchoring for the whole disc achieved through the fixation of the flanges to the vertebrae with screws, bone anchors or a similar type of fixation system is strong. It may be possible, in alternative embodiments to provide a more "free floating" device with the annulus of the disc sutured closed around the device to prevent migration.

Thirdly, the material of the outer jacket can be configured to give the desired structural properties, whilst also providing a relatively open structure for the material. This assists in providing good conditions for tissue ingrowth, both through the outer jacket and eventually through the inner jacket. The outer jacket can provided the desired access, but also act as a scaffold. As with the inner jacket, various combinations of bio-absorbable and non-absorbable materials can be used to assist this process.

The use of an inner jacket and outer jacket is also beneficial in that the use of multiple jackets allows the proportion of embroidery to elastomer to remain similar to that established as beneficial in the cervical disc.

In designing the artificial lumbar disc the aim has been to provide a disc having appropriate compressive stiffness. The decompression of the spinal cord through the opening of the disc space is one of the key principles in the relief of pain through disc replacement or fusion. To achieve this the artificial disc is provided with a compressive stiffness curve (force against displacement) similar or higher to the natural disc it is intended to replace. The properties of the core can be modified by doping or the like. For instance, the core may be provided with 13% barium sulphate.

Ideally, the artificial disc mimics as many of the motion stiffnesses as possible of a natural disc. Flexion/extension motions are both the most common and the largest (in terms of angle) motions that occur in the lumbar spine. This is the key stiffness which the above artificial disc seeks to match. The ability to carry shear and torsional loads on the disc itself should help protect the facet joints and is therefore also mimicked as far as possible.

One of the intentions with disc prostheses of the above mentioned type and type described in US6093205 is to encourage tissue ingrowth into the disc prosthesis. The ingrowth of such soft tissue into the outer jacket and/or inner jacket and/or flanges may occur. The benefit of this is that biological fixation of the prosthesis in the disc space occurs in the long term and this in turn resists undesirable migration of the prosthesis out of the correct position within the disc space. The flanges and the anchoring they provide are particularly useful in this context as they provide secure fixation of the prosthesis whilst this biological fixation develops over the first few months after implantation. The flanges may also provide a useful scaffold for the development of a biological anterior longitudinal ligament.

Whilst the flanges need to provide a high level of fixation during the first few months after implantation, once ingrowth has occurred this level of fixation is not needed. As a result, the level of tension in the flanges needed to give fixation may be undesirably high in the long term as it resists the full extension range of the spine. This is particularly a potential issue for optimum performance in the case of neck disc prostheses , where the extension range is greater.

To address this issue and provide still further improved disc prostheses, designs have been developed which reduce the tension in the flanges a few months after implantation. This may be through a reduction in the tension or its removal through the detachment of the flanges. As a result, once the biological fixation has had time to develop under preferred conditions and with mechanical restraint of the prosthesis, the prosthesis allows the full range of movement and does not compromise the spines operation long term.

A number of designs suitable for general use in the spine, including lumbar and cervical disc spaces have been developed.

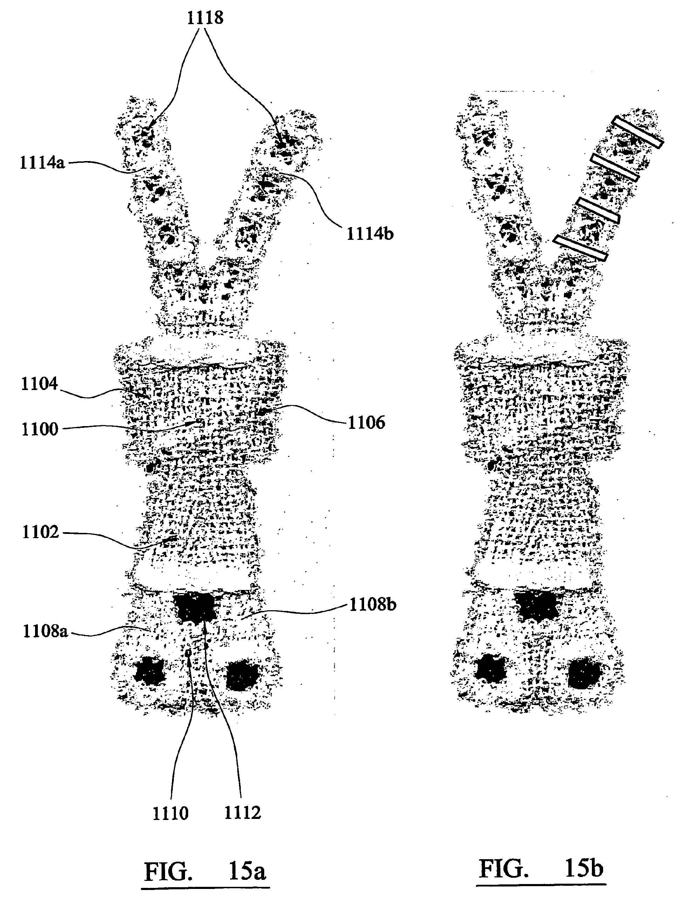

Referring to Figure 15a, an outer jacket in its flat form, before assembly to surround the core, is shown. The core would be surrounded by bottom wall 1100, by the two side walls 1104 and 1106 attached to the bottom wall 1100 and by the top wall 1102. A first pair of flanges 1108a, 1108b extend from the top wall 1102 and are joined together by a web 1110. The web 1110 and flanges 1108a, 1108b define the bounds of a hole 1112. The second pair of flanges 1114a, 1114b are attached to the bottom wall 1100 and in use are passed through the hole 1112 to provide the above mentioned interdigitation. The ends of the flanges 1108a and 1108b both have apertures 1116 which accommodate fixing screws inserted into the spine in use. The ends of the flanges 1114a, 1114b, could be provided with such apertures for fixing screws, but in this case are provided with sections 1118 for receiving sutures, not shown. The operation of this feature is described in more detail below, and of course such a structure could be used in the case of both flange pairs as the fixing.

In a first design approach, the flanges are joined to the rest of the outer jacket which encloses the core by a zone of different material. This different material is made of an absorbable fibre and as a consequence, after the desired controlled period, the zone disappears and so ceases to join the flanges to the outer jacket for the core anymore. As a result, the tension in provided by the flanges is released and the full range of extension is provided. The absorption process would preferably be gradual so as to provide a phase reduction in the tension and hence phased increase in the range of movement.

In a second design approach, the flanges are formed from at least two different material. The flanges include load bearing fibres, which are placed under and maintain the desired tension, and other fibres. The load bearing fibres are made of an absorbable fibre and as a consequence, after the desired controlled period, they are absorbed and so are no longer available to bear the load and the tension is released. The other fibres are intended to be permanent and so are then all that remains of the flanges. These other fibres may serve still to define the overall shape of the flanges, maintain the interdigitation and potentially maintain a reduced level of tension. At least a slackening of the tension results and an increased or even full range of extension is provided. The absorption process would again preferably be gradual so as to provide a phase reduction in the tension and hence phased increase in the range of movement.

In a third design, the flanges include fibres which assume a zigzag path away from the rest of the outer jacket which holds the core and towards the ends of the flanges. When implanted, the zigzag path these fibres take is maintained because these fibres are not subjected to the load applied to the flanges. Instead, that load is borne by other fibres which are attached to the outer jacket and fixation locations. These other fibres are bio-absorbable and so with time disappear. The result is that the load transfers from the other fibres to the zigzag fibres and the zigzag fibres straighten. The result is a slackening of the tension in the flanges and an increase in the range of extension possible.

In a fourth design, the zigzag fibres are again used, but this time together with a series of fibres which bridge the zigzags. The bridging fibres may be stuck to the zigzag fibres and/or wound round them and/or connected to the zigzag fibres in a fixed manner. The overall result is that these bridging fibres prevent the zigzags opening up to a linear form, at the time of implantation, and so prevent the flanges extending, when the desired tension is applied. As the bridging fibres disappear, the load transfers to the zigzag fibres, they straighten, the tension slackens and the extension range for the spine is increased.

In each of these designs, the use of sets of materials in the prostheses means that the transition is made gradual. For instance, slightly different materials and/or different diameters and/or dimensions and/or densities of absorbable material can be used so as to give different periods before each of those different materials is predominantly absorbed and so ceases to bear loads. Slightly different materials could also be used to vary the extent of tissue ingrowth experienced by different parts of the prosthesis, and particularly within different parts of the flanges, between zero and the maximum possible. Zero growth may be desirable where in growth is of no real benefit, for instance in locations where the release of tension would soon render it redundant. Avoiding in-growth in these areas may increase the extent of in-growth where it is beneficial. In-growth may be prevented through the use of appropriate materials to define the fixing locations, for instance. Ultra-high molecular weight polyethylene may be used as such a material.

The ends of the flanges, as mentioned briefly above, are provided with sections 1118 for receiving sutures. Such an arrangement could be provided for the ends of both pairs of flanges. These sections are formed of a reinforced parts 1120 which extend across the flanges between the load bearing fibres 1122 on one side of the flange and the load bearing fibres 1122 on the other side of the flange. A series of such reinforced parts 1120 are provided spaced along the length of the flanges. Between the reinforced parts 1120 are mesh parts 1124 forming openings which are criss-crossed by a series of fibres. These mesh parts 1124 allow the suture to be readily positioned by wrapping it around the reinforced parts 1120. By providing a series of alternating mesh parts 1124 and reinforced parts 1122 along the flanges a variety of fixing locations for use in attaching to the spine are provided.

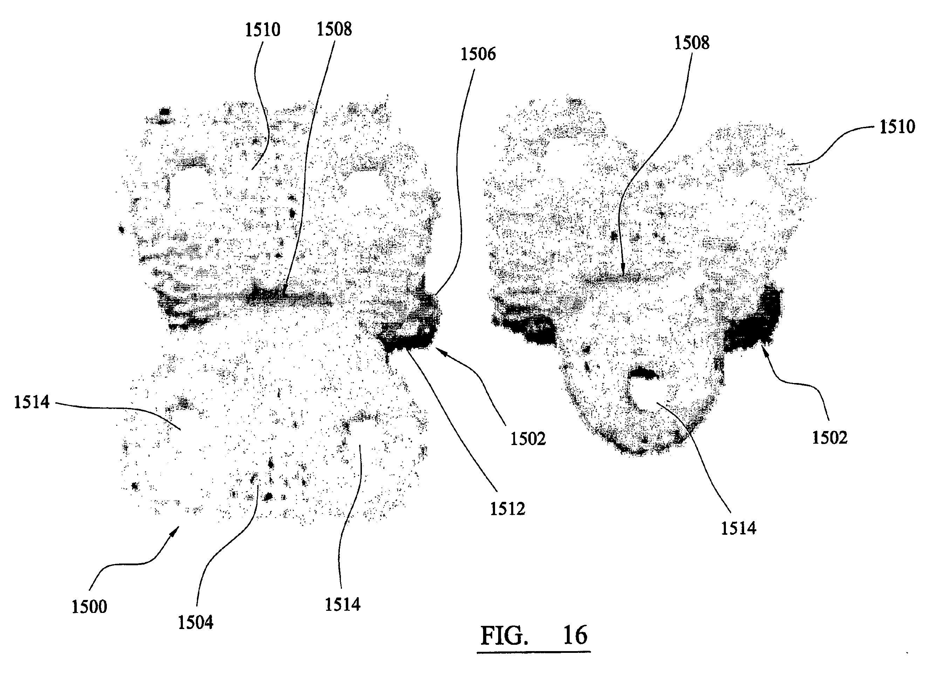

Figure 16 shows, left hand side, an outer jacket 1500 of one form of the present invention. The body 1502 of the outer jacket 1500 surrounds the core. The flange 1504 extending from the top surface 1506 of the body 1502 passes down through a hole 1508 in the flange 1510 extending from the bottom surface 1512 of the body 1502. The resulting interdigitation closes off the opening in the body 1502 which allows the core to be introduced. Each flange 1504, 1510 is provided with two holes 1514 which receiving fixings to attach the flanges to the spine.

In an another form, Figure 16 right hand side, the body 1502 and lower flange 1510 extending from it are provided in the same way as the left hand side form described above. The difference lies in the configuration of the other flange 1520. Again this flange 1520 is interdigitated with the flange 1510 by being passed through a hole 1508 in the flange 1510. The flange 1520 is provided with a single hole 1514 which receives a fixing. However, the flange 1520 does not flare out to as great a width as the flange 1504 in the left hand side form. This results in a generally Y-shaped profile presented by the parts of the flanges 1510, 1520 extending beyond the location of interdigitation.

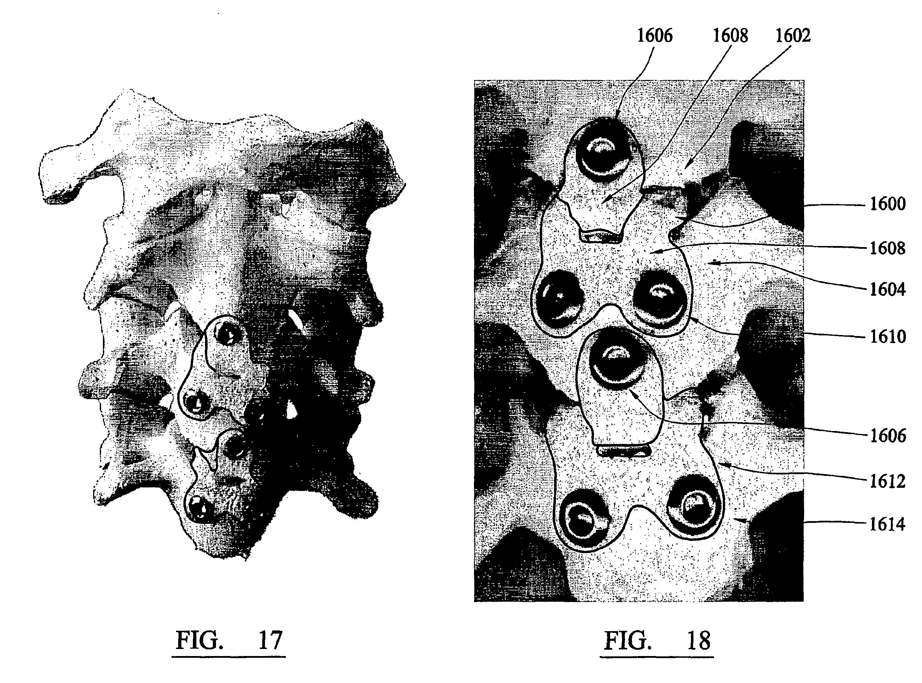

The benefits of the Y-shaped profile are explained with reference to Figure 17 and Figure 18. One assembled artificial disc 1600 is inserted between a first vertebra 1602 and a second vertebra 1604. The artificial disc 1600 is fixed to the first vertebra 1602 by virtue of a fixing 1606 which passes through the hole in the flange 1608. The head of the fixing 1606 is larger than the hole in the flange 1608 it passes through so giving a secure fixing to the vertebra 1602. The artificial disc 1600 is fixed to the second vertebra 1604 by virtue of two fixings 1610. Thus the stem of the Y-shaped profile is fixed to the first vertebra 1602, whilst the fork of the Y-shaped profile is fixed to the second vertebra 1604.

A second assembled artificial disc 1612 is inserted between a third vertebra 1614 and the second vertebra 1604. The second artificial disc 1612 is provided with the Y-shaped profile in the same orientation. Thus the fork of the Y-shaped profile is fixed to the third vertebra 1614, whilst the stem of the Y-shaped profile is fixed to the second vertebra 1604. This means that the second vertebra 1604 need only accommodate one fixing 1606 from the second artificial disc 1612 and two from the first artificial disc 1600, with those fixings in different positions across the face of the second vertebra 1604. This means that the fixings take up less room because of the lower number used, at even less room because of the different positions they occupy. The central fixing 1606 of the second artificial disc 1612 can be nested between the fixings 1610 of the first artificial disc 1600.

The nesting or interlocking nature of disc flanges provided in this way enable artificial discs to be provided at adjacent levels along this spine. This arrangement is particularly useful in the context of the cervical part of the spine where space is limited. As well as using a reduced number of fixings, this form of flanges also avoids overlapping of the flange from one disc replacement with the flange of another. Overlapping material is undesirable as it increases the space occupied by the replacement disc on the anterior face of the spine and renders the replacement less minimal. The flanges of the disc replacement still provided the desired anterior longitudinal ligament replacement. The fixings still provide the desired torsional stability. This type of artificial disc is still useful where only a single disc replacement is needed, however.

## Claims

1. A disc prosthesis comprising:
a core (116), the core being of elastomeric material, the core being provided within an inner component (118), the inner component being of fabric, the inner component being provided within an outer component (120), the outer component being of fabric,
**characterized in that** the inner component (118) provides a smooth inner contact surface for the core (116), and movement between the inner (118) and outer (120) components is facilitated in preference to movement between the inner component (118) and core (116).

2. The disc prosthesis according to claim 1, wherein the core is a single elastomeric component.

3. The disc prosthesis according to claim 1, wherein the core is formed of multiple elastomeric components, with each provided within its own inner component.

4. The disc prosthesis according to claim 1, wherein the core provides a planar top surface and planar lower surface, the top and bottom surfaces not being parallel to one another, the separation of the top and bottom surfaces increasing from one side of the core to the other.

5. The disc prosthesis according to claim 1, wherein at least one of the top surface and bottom surface of the core is at least one of octagonal, hexagonal, round, and elliptic.

6. The disc prosthesis according to claim 1, wherein any movement, particularly sliding movement, within the disc is greater between the outer component and inner component than between the inner component and core.

7. The disc prosthesis according to claim 1, wherein the inner component is at least one of configured to and formed of one or more materials intended to promote tissue growth.

8. The disc prosthesis according to claim 1, wherein one or more materials used in the inner component are bio-absorbable.

9. The disc prosthesis according to claim 1, wherein said smooth inner contact surface provides uniform contact between the inner surface of the inner component and the core.

10. The disc prosthesis according to claim 1, wherein a top wall of the inner component is connected to a side wall and hence to a bottom wall, with one or more further side walls being connected to at least one of the top wall, side wall, and bottom wall.

11. The disc prosthesis according to claim 10, wherein the inner component is formed from an element including a side wall connected on one edge to a top wall and connected on an opposing edge to a bottom wall, the side wall being connected on one side edge to one other side wall and the side wall being connected on the other side edge to one or more other walls.

12. The disc prosthesis according to claim 10, wherein the
side walls of the inner component are contacted additional elements, provided by a continuous band extending around the side of the inner component.

13. The disc prosthesis according to claim 1, wherein the outer component is at least one of configured and formed of one or more materials intended to promote tissue growth, particularly tissue ingrowth at least one of through the outer component, between the inner component and the core, and through the inner component.

14. The disc prosthesis according to claim 1, wherein one or more materials used in the outer component are bio-absorbable.

15. The disc prosthesis according to claim 1, wherein the outer component is formed from an element including a side wall connected on one edge to a top wall and connected on an opposing edge to a bottom wall, the side wall being connected on one side edge to two other side walls, the side wall being connected on the other side edge to two other side walls, a further side wall being connected to the opposite edge of the top wall or bottom wall to the edge to which the side wall linking the top wall and bottom wall is provided.

16. The disc prosthesis according to claim 15, wherein one or more edges of at least one of the top wall and the bottom wall of the outer component are provided with flanges, the flanges providing anchor locations for attaching the outer component to one or more vertebrae.

## Patentansprüche

1. Bandscheibenprothese, die Folgendes umfasst:
einen Kern (116), wobei der Kern aus elastomerem Material besteht und innerhalb einer inneren Komponente (118) vorgesehen ist, wobei die innere Komponente aus Gewebe besteht und innerhalb einer äußeren Komponente (120) vorgesehen ist, wobei die äußere Komponente aus Gewebe besteht,
**dadurch gekennzeichnet, dass** die innere Komponente (118) eine glatte innere Kontaktfläche für den Kern (116) bereitstellt und dass eine Bewegung bevorzugter zwischen den inneren (118) und äußeren (120) Komponenten als zwischen der inneren Komponente (118) und dem Kern (116) ermöglicht wird.

2. Bandscheibenprothese nach Anspruch 1, bei der der Kern eine einzelne elastomere Komponente ist.

3. Bandscheibenprothese nach Anspruch 1, bei der der Kern aus mehreren elastomeren Komponenten gebildet ist, die jeweils innerhalb ihrer eigenen inneren Komponente vorgesehen sind.

4. Bandscheibenprothese nach Anspruch 1, bei der der Kern eine planare obere Fläche und eine planare untere Fläche bereitstellt, wobei die oberen und unteren Flächen nicht parallel zueinander verlaufen und die Trennung der oberen und unteren Flächen von einer Seite des Kerns zur anderen zunimmt.

5. Bandscheibenprothese nach Anspruch 1, bei der mindestens die obere Fläche oder die untere Fläche des Kerns mindestens oktagonal, hexagonal, rund oder elliptisch ausgebildet ist.

6. Bandscheibenprothese nach Anspruch 1, bei der eine beliebige Bewegung, insbesondere eine gleitende Bewegung, innerhalb der Scheibe zwischen der äußeren Komponente und der inneren Komponente größer als zwischen der inneren Komponente und dem Kern ist.

7. Bandscheibenprothese nach Anspruch 1, bei der die innere Komponente mindestens für ein oder mehrere Materialien, die Gewebewachstum fördern sollen, ausgelegt und daraus gebildet ist.

8. Bandscheibenprothese nach Anspruch 1, bei der ein oder mehrere Materialien, die in der inneren Komponente Verwendung finden, bioresorbierbar sind.

9. Bandscheibenprothese nach Anspruch 1, bei der die glatte innere Kontaktfläche für einen gleichmäßigen Kontakt zwischen der inneren Fläche der inneren Komponente und dem Kern sorgt.

10. Bandscheibenprothese nach Anspruch 1, bei der eine obere Wand der inneren Komponente mit einer Seitenwand und somit mit einer unteren Wand verbunden ist, wobei eine oder mehrere weitere Seitenwände mindestens mit der oberen Wand, der Seitenwand oder der unteren Wand verbunden sind.

11. Bandscheibenprothese nach Anspruch 10, bei der die innere Komponente aus einem Element gebildet ist, das eine Seitenwand beinhaltet, die an einer Kante mit einer oberen Wand und an einer gegenüberliegenden Kante mit einer unteren Wand verbunden ist, wobei die Seitenwand an einer Seitenkante mit einer anderen Seitenwand und an der anderen Seitenkante mit einer oder mehreren anderen Wänden verbunden ist.

12. Bandscheibenprothese nach Anspruch 10, bei der die Seitenwände der inneren Komponente in Berührung stehende zusätzliche Elemente sind, die durch ein fortlaufendes Band bereitgestellt werden, das um die Seite der inneren Komponente herum verläuft.

13. Bandscheibenprothese nach Anspruch 1, bei der die äußere Komponente mindestens für ein oder mehrere Materialien ausgelegt und daraus gebildet ist, die Gewebewachstum, insbesondere ein Einwachsen von Gewebe mindestens durch die äußere Komponente, zwischen der inneren Komponente und dem Kern oder durch die innere Komponente, fördern sollen.

14. Bandscheibenprothese nach Anspruch 1, bei der ein oder mehrere Materialien, die in der äußeren Komponente Verwendung finden, bioresorbierbar sind.

15. Bandscheibenprothese nach Anspruch 1, bei der die äußere Komponente aus einem Element gebildet ist, das eine Seitenwand beinhaltet, die an einer Kante mit einer oberen Wand und an einer gegenüberliegenden Kante mit einer unteren Wand verbunden ist, wobei die Seitenwand an einer Seitenkante mit zwei anderen Seitenwänden und an der anderen Seitenkante mit zwei anderen Seitenwänden verbunden ist, und wobei eine weitere Seitenwand an der gegenüberliegenden Kante der oberen Wand oder der unteren Wand mit derjenigen Kante verbunden ist, an der die die obere Wand und die untere Wand verbindende Seitenwand vorgesehen ist.

16. Bandscheibenprothese nach Anspruch 15, bei der eine oder mehrere Kanten von mindestens der oberen Wand oder der unteren Wand der äußeren Komponente mit Flanschen versehen sind, wobei die Flansche Ankerstellen bereitstellen, um die äußere Komponente an einem oder mehreren Wirbeln befestigen zu können.

## Revendications

1. Prothèse de disque, comprenant :
un coeur (116), le coeur étant en matériau élastomère, le coeur étant prévu dans un composant interne (118), le composant interne étant en tissu, le composant interne étant prévu dans un composant externe (120), le composant externe étant en tissu,
**caractérisée en ce que** le composant interne (118) offre une surface de contact interne lisse pour le coeur (116) et le mouvement entre le composant interne (118) et le composant externe (120) est facilité préférablement au mouvement entre le composant interne (118) et le coeur (116).

2. Prothèse de disque selon la revendication 1, dans laquelle le coeur est un composant élastomère unique.

3. Prothèse de disque selon la revendication 1, dans laquelle le coeur est formé de composants élastomères multiples, chacun étant pourvu de son propre composant interne.

4. Prothèse de disque selon la revendication 1, dans laquelle le coeur offre une surface supérieure plane et une surface inférieure plane, les surfaces supérieure et inférieure n'étant pas parallèles l'une à l'autre, la séparation entre les surfaces supérieure et inférieure augmentant d'un côté du coeur à l'autre.

5. Prothèse de disque selon la revendication 1, dans laquelle au moins l'une de la surface supérieure et de la surface inférieure du coeur est au moins octogonale, hexagonale, ronde, et/ou elliptique.

6. Prothèse de disque selon la revendication 1, dans laquelle tout mouvement, en particulier tout mouvement de glissement dans le disque est plus important entre le composant externe et le composant interne qu'entre le composant interne et le coeur.

7. Prothèse de disque selon la revendication 1, dans laquelle le composant interne est au moins un composant configuré pour, ou formé d'un ou plusieurs matériaux destinés à, favoriser la croissance tissulaire.

8. Prothèse de disque selon la revendication 1, dans laquelle un ou plusieurs des matériaux utilisés dans le composant interne sont bio-absorbables.

9. Prothèse de disque selon la revendication 1, dans laquelle ladite surface de contact interne lisse permet un contact uniforme entre la surface interne du composant interne et le coeur.

10. Prothèse de disque selon la revendication 1, dans laquelle une paroi supérieure du composant interne est connectée à une paroi latérale et donc à une paroi inférieure, une ou plusieurs autres parois latérales étant raccordées à au moins l'une de la paroi supérieure, la paroi latérale ou la paroi inférieure.

11. Prothèse de disque selon la revendication 10, dans laquelle le composant interne est formé d'un élément comportant une paroi latérale raccordée au niveau d'un bord à une paroi supérieure et raccordée au niveau d'un bord opposé à une paroi inférieure, la paroi latérale étant raccordée au niveau d'un bord latéral à une autre paroi latérale et la paroi latérale étant raccordée au niveau de l'autre bord latéral à une ou plusieurs autres parois.

12. Prothèse de disque selon la revendication 10, dans laquelle les parois latérales du composant interne sont mises en contact des éléments additionnels constitués par une bande continue s'étendant tout autour du côté du composant interne.

13. Prothèse de disque selon la revendication 1, dans laquelle le composant externe est au moins un composant configuré et formé d'un ou plusieurs matériaux destinés à promouvoir la croissance tissulaire, en particulier la croissance tissulaire intérieure au moins à travers le composant externe, entre le composant interne et le coeur, ou à travers le composant interne.

14. Prothèse de disque selon la revendication 1, dans laquelle un ou plusieurs matériaux utilisés dans le composant externe sont bio-absorbables.

15. Prothèse de disque selon la revendication 1, dans laquelle le composant externe est formé d'un élément comportant une paroi latérale raccordée au niveau d'un bord à une paroi supérieure et raccordée au niveau d'un bord opposé à une paroi inférieure, la paroi latérale étant raccordée au niveau d'un bord latéral à deux autres parois latérales, la paroi latérale étant raccordée au niveau de l'autre bord latéral à deux autres parois latérales, une paroi latérale supplémentaire étant raccordée au bord opposé de la paroi supérieure ou de la paroi inférieure au bord auquel la paroi latérale raccordant la paroi supérieure et la paroi inférieure est prévue.

16. Prothèse de disque selon la revendication 15, dans laquelle un ou plusieurs bords d'au moins une de la paroi supérieure ou de la paroi inférieure du composant externe sont pourvus de brides, les brides offrant des emplacements d'ancrage pour attacher le composant externe à une ou plusieurs vertèbres.
